(19) Europäisches Patentamt
European Patent Office
Office européen des brevets

(11) Publication number: 0 496 496 B1

# EUROPEAN PATENT SPECIFICATION

(45) Date of publication of patent specification: 27.12.95

(51) Int. Cl.⁶: **B01D 61/36**, B01D 69/12, C07C 29/76

(21) Application number: 92300125.9

(22) Date of filing: 07.01.92

(54) Membrane dehydration process

(30) Priority: 18.01.91 US 642938

(43) Date of publication of application:
29.07.92 Bulletin 92/31

(45) Publication of the grant of the patent:
27.12.95 Bulletin 95/52

(84) Designated Contracting States:
DE FR GB IT NL

(56) References cited:
EP-A- 0 425 108
US-A- 4 659 590

WORLD PATENTS INDEX LATEST Section Ch,
Week 8828, Derwent Publications Ltd., London, GB; Class A, AN 88-193436

WORLD PATENTS INDEX LATEST Section Ch,
Week 8302, Derwent Publications Ltd., London, GB; Class A, AN 83-28537K

WORLD PATENTS INDEX LATEST Section Ch,
Week 8419, Derwent Publications Ltd., London, GB; Class A, AN 84-117457

WORLD PATENTS INDEX LATEST Section Ch,

Week 8419, Derwent Publications Ltd., London, GB; Class A, AN 84-117456

(73) Proprietor: TEXACO DEVELOPMENT CORPORATION
2000 Westchester Avenue
White Plains,
New York 10650 (US)

(72) Inventor: Reale, John, Jr.
8 Sidney Lane
Wappingers Falls,
New York 12590 (US)

(74) Representative: Green, Mark Charles et al
Urquhart-Dykes & Lord,
91 Wimpole Street
London W1M 8AH (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to a process for the dehydration of organic oxygenates such as isopropyl alcohol or methyl ethyl ketone. More particularly it relates to a membrane technique for effecting separation of water from an aqueous mixture containing isopropyl alcohol or methyl ethyl ketone. The invention further relates to a membrane for use therein.

As well known to those skilled in the art, it is possible to remove water from mixtures thereof with organic liquids by various techniques including adsorption or distillation. These conventional processes, particularly distillation, are however, characterized by high capital cost. In the case of distillation for example the process requires expensive distillation towers, heaters, heat exchangers (reboilers, condensers) together with a substantial amount of auxiliary equipment typified by pumps, collection vessels, vacuum generating equipment.

Such operations are characterized by high operating costs principally costs of heating and cooling - plus pumping.

Furthermore the properties of the materials being separated, as is evidenced by the distillation curves, may be such that a large number of plates may be required, etc. When the material forms an azeotrope with water, additional problems may be present which for example, would require that separation be effected in a series of steps (e.g. as in two towers) or by addition of extraneous materials to the system.

There are also comparable problems which are unique to adsorption systems.

It has been found to be possible to utilize membrane systems to separate mixtures of miscible liquids by pervaporation. In this process, the charge liquid is brought into contact with a membrane film; and one component of the charge liquid preferentially permeates the membrane. The permeate is then removed as a vapor from the downstream side of the film - typically by sweeping with a carrier gas or by reducing the pressure below the saturated vapor pressure of the permeating species.

Illustrative membranes which have been employed in prior art techniques include those set forth in the following list:

| Separating Layer | References |
|---|---|
| – Nafion(TM) brand of perfluorosulfonic acid | – Cabasso and Liu J. Memb. Sci. 24, 101 (1985) |
| – Sulfonated polyethylene | – Cabasso, Korngold & Liu J. Pol. Sc: Letters, 23, 57 (1985) |
| – Fluorinated polyether or Carboxylic Acid fluorides | – USP 4,526,948 to Dupont |

TABLE

| Separating Layer | References |
| --- | --- |
| - Selemion™ AMV brand of Asahi Glass cross-linked styrene butadiene (with quaternary ammonium residues on a polyvinyl chloride backing) | - Wentzlaff Boddeker & Hattanbach J. Memb. Sci. 22,333 (1985) |
| - Cellulose triacetate | - Wentzlaff, Boddeker & Hattanback, J. Memb. Sci. 22, 333 (1985) |
| - Polyacrylonitrile | - Neel, Aptel & Clement Desalination 53, 297 (1985) |
| - Crosslinked Polyvinyl Alcohol | - EP-A - 0 096 339 (GFT) |
| - Poly(maleimideacrylonitrile) | - Yoshikawa et al J. Pol. Sci. 22, 2159 (1984) |
| - Dextrine -isophorone diisocyanate | - Chem. Econ. Eng. Rev., Vol. 17, No. 12, page 34 (1985) |

The cost effectiveness of a membrane is determined by the selectivity and productivity. Of the membranes commercially available, an illustrative polyvinyl alcohol membrane of high performance is that disclosed in EP-A-0 096 339.

EP-A-0096339 discloses as cross-linking agents, diacids (typified by maleic acid or fumaric acid); dihalogen compounds (typified by dichloroacetone or 1,3-dichloroisopropanol); aldehydes, including dialdehydes, typified by formaldehyde. These membranes are said to be particularly effective for dehydration of aqueous solutions of ethanol or isopropanol.

This reference discloses separation of water from alcohols, ethers, ketones, aldehydes, or acids by use of composite membranes. Specifically the composite includes (i) a backing typically about 120 $\mu$m in thickness, on which is positioned (ii) a microporous support layer of a polysulfone or a polyacrylonitrile of about 50 $\mu$m thickness, on which is positioned (iii) a separating layer of cross-linked polyvinyl alcohol about 2 $\mu$m in thickness.

Polyvinyl alcohol may be cross-linked by use of difunctional agents which react with the hydroxyl group of the polyvinyl alcohol. Typical cross-linking agent may include dialdehydes (which yield acetal linkages), diacids or diacid halides (which yield ester linkages), dihalogen compounds or epichlorhydrin (which yield ether linkages) olefinic aldehydes (which yield ether/acetal linkages), boric acid (which yields boric ester linkages), sulfonamidoaldehydes, etc.

JP-A-59055304, JP-A-59055305, JP-A-63130105 and US-A-4659590 describe a membrane comprising a polyethylene imine separating layer which has been chemically crosslinked with a crosslinking agent followed by a heat curing and a method for concentrating an aqueous charge mixture of organic oxygenate (isopropanol or ethanol) by pervaporation using said membrane. This crosslinking agent is an aromatic compound having acid halide and/or isocyanate groups (e.g. toluene diisocyanate) in JP-A-59055304, an aromatic compound having acid halide and/or sulfonyl halide groups (e.g. chlorosulfonyl isophthaloyl chloride) in JP-A-59055305, a compound having acid halide, isocyanate, sulfonyl halide, epoxy or acid anhydride groups (e.g. toluene diisocyanate) in JP-A-63130105, and toluene diisocyanate, glyoxal or glutaric aldehyde in US-A-4659590.

The present invention is directed to a process which comprises

passing a charge containing water and an organic oxygenate into contact with, as a separation membrane, a non-porous polyimine polymer separating layer which has firstly been chemically cross-linked with an aliphatic polyhalide cross-linking agent, and which has secondly been thermally crosslinked ;

maintaining a pressure drop across said membrane thereby forming a high pressure retentate containing increased content of organic oxygenate and decreased content of water and a lower pressure permeate containing increased content of water and decreased content of organic oxygenate;

maintaining pressure on the high pressure retentate above the vapor pressure of said retentate thereby maintaining the high pressure retentate in liquid phase;

maintaining the pressure on the low pressure permeate below the vapor pressure of said permeate thereby maintaining the low pressure permeate in vapor phase

The retentate may then be recovered from the high pressure side of said non-porous separating layer said high pressure retentate in liquid phase containing increased content of organic oxygenate and decreased content of water.

The permeate may be recovered from the low pressure side of said non-porous separating layer, said low pressure permeate in vapor phase containing increased content of water and decreased content of organic oxygenate.

The invention further relates to a non-porous membrane comprising a separating layer of a polyimine polymer which has firstly been cross-linked with an aliphatic polyhalide and which has secondly been thermally crosslinked.

## THE MEMBRANE ASSEMBLY

The process of this invention may be carried out by use of a composite structure which in one preferred embodiment may include (i) a carrier layer which provides mechanical strength, (ii) a porous support layer, and (iii) a separating layer across which separation occurs.

The composite structure of this invention includes a multi-layer assembly which in the preferred embodiment preferably includes a porous carrier layer which provides mechanical strength and support to the assembly.

## THE CARRIER LAYER

This carrier layer, when used, is characterized by its high degree of porosity and mechanical strength. It may be fibrous or non-fibrous, woven or non-woven. In the preferred embodiment, the carrier layer may be a porous, flexible, woven fibrous polyester. A typical polyester carrier layer may be formulated of non-woven, thermally-bonded strands.

A preferred non-woven polyester carrier layer may be formulated of non-woven, thermally-bonded strands and characterized by a fabric weight of 80 ± 8 grams per square yard (96 ± 9.6g/m$^2$), a thickness of 4.2 ± 0.5 mils (106.7 ± 12.7 $\mu$m), a tensile strength (in the machine direction) of 31 psi (213 kPa) and (in cross direction) of 10 psi (69 kPa) and a Frazier air permeability of 6 cuft/min/sq. ft. @ 0.5 inches of water (182 cm$^3$/min/cm$^2$ @ 125 Pa).

## THE POROUS SUPPORT LAYER

The porous support layer (typically an ultrafiltration membrane) which may be used in practice of this invention is preferably formed of polyacrylonitrile polymer. Typically the polyacrylonitrile may be of thickness of 40-80 $\mu$m , say 50 $\mu$m and is preferably characterized by a pore size of less than about 500A and typically about 200A. This corresponds to a molecular weight cut-off of less than about 100,000, typically about 40,000.

A preferred porous support layer may be the Daicel DUY-L™ polyacrylonitrile of 40,000 molecular weight cut-off.

Typically the support layer may be characterized by a molecular weight $\overline{M}_n$ of 100,000, a $T_m$ of 319°C, a $T_g$ of 85°C, a decomposition temperature of 250°C, a tensile strength at yield of 250-568 MPa, a Linear Thermal mansion Coefficient of 1.6 K$^{-1}$ (above $T_g$) and of 1.0 K$^{-1}$ (below $T_g$), and Water Absorption (at 21°C and 65% relative humidity) of 1-2.5%. ($T_m$ is the melting point and $T_g$ is the glass transition temperature).

## THE SEPARATING LAYER

The separating layer which permits attainment of separation in accordance with the process of this invention includes a thermally treated non-porous film or membrane of 0.2-5 $\mu$m say about 2 $\mu$m of a polyimine polymer of molecular weight $\overline{M}_n$ of about 40,000-100,000, say about 60,000 (prior to cross-linking), which is chemically cross-linked by an aliphatic polyhalide cross-linking agent. The chemically cross-linked membranes (i) may permit attainment of higher flux and higher selectivity, (ii) may permit attainment of higher selectivity at comparable flux, (iii) may permit attainment of higher flux at comparable selectivity, and (iv) may permit attainment of a membrane which is more resistant to deterioration.

Polyimine polymers may be characterized by the presence of recurring -N-R''- groups as integral parts of the main polymer chain. Typical structural formula of linear polyimines may be represented as

H$_2$N-R''[N-R'']$_n$ -NH$_2$

wherein $\underline{n}$ represents the degree of polymerization or number of recurring groups in the polymer chain.

In the above formula, R'' may be a hydrocarbon group selected from alkylene, aralkylene, cycloalkylene, arylene, and alkarylene, including such radicals when inertly substituted. When R'' is alkylene, it may typically be methylene, ethylene, n-propylene, iso-propylene, n-butylene, i-butylene, sec-butylene, amylene, octylene, decylene, octadecylene. When R'' is aralkylene, it may typically be benzylene, beta-phenylethylene. When R'' is cycloalkylene, it may typically be cyclohexylene, cycloheptylene, cyclooctylene, 2-methylcycloheptylene, 3-butylcyclohexylene, 3-methylcyclohexylene. When R'' is arylene, it may typically be phenylene, naphthylene. When R''is alkarylene, it may typically be tolylene, xylylene. R'' may be inertly substituted i.e. it may bear a non-reactive substitutent such as alkyl, aryl, cycloalkyl,ether. Typically inertly substituted R'' groups may include 3-methoxyropylene, 2-ethoxyethylene, carboethoxymethylene, 4-methylcyclohexylene, p-methylphenylene, p-methylbenzylene, 3-ethyl-5-methylphenylene. The preferred R'' groups may be phenylene or lower alkylene, i.e. C1-C10 alkylene, groups including e.g. methylene, ethylene, n-propylene, i-propylene, butylene, amylene, hexylene, octylene, decylene. R'' may preferably be phenylene or ethylene $-CH_2CH_2-$.

Illustrative polyimine polymers include those of molecular weight $\overline{M}_n$ of 40,000-100,000, say 60,000.

Suitable polyimines may include the following, the first listed being preferred:

## TABLE

A. Cordova Chemical Company Corcat P-600 brand of polyethyleneimine resin membrane ($\overline{M}_n$ of 60,000) in 33 w% aqueous solution - Brookfield viscosity @ 25°C of 5000 cP, Sp.Gr @ 25°C of 1.04-1.06, and pH of 10-11, having the formula

$$R(NCH_2CH_2)_n \; NH_2$$
$$|$$
$$R$$

wherein R is H or $(CH_2CH_2N)_x$ (containing 30% primary, 40% secondary, and 30% tertiary amines).

B. Dow Chemical Co Tydex 12 brand of polyethyleneimine membrane ($\overline{M}_n$ of 50,000) in 30w% aqueous solution having the same formula as the Corcat P-600 membrane.

The chemical cross-linking agents will contain an aliphatic moiety, preferably containing 2-12 carbon atoms, typically 3-6 carbon atoms, say 4 carbon atoms. The cross-linking agent will be a polyhalide, and typically contains 2-5 halogen atoms, most preferably 2. The halogen is preferably bromine or less preferably chlorine or iodine. The halides may preferably be alpha, omega dihalides of linear straight chain aliphatic hydrocarbon. Typical cross-linking agents may be as tabulated below, the first listed being preferred:

1,4-dibromo-n-butane (DBB)
1,5-dibromo-n-pentane (DBP)
1,3-dibromo propane
1,6-dibromo hexane
1,8-dibromo octane
1,4-dichloro-n-butane

The separating layer may be prepared by cross-linking a polyimine polymer in situ.

In situ cross-linking may be carried out by casting onto the preferred polyacrylonitrile support the polyimine polymer typically in solution in butanol to which has been added the cross-linking agent (typically 1, 4-dibromo-butane) in mole ratio of cross-linking agent to polymer of 0.1-3, say about 1.67.

It may be possible in one embodiment to cross-link the polyimine separating layer in one step by casting the solution of polyimine polymer and polyhalide, followed by heat curing the cast membrane.

The heat curing step at 100°C-200°C, say 125°C for 1-30 minutes, say 7 minutes effects a cross-linking of the non-porous polyimine separating layer. After using a chemical cross-linking agent, such as 1,4-dibromo-butane, the thermal treating augments and accelerates the cross-linking.

## THE COMPOSITE MEMBRANE

It is a feature of this invention that the composite membrane of this invention may comprise (i) an optional carrier layer, characterized by porosity and mechanical strength, for supporting a porous support layer and a separating layer, (ii) a porous support layer of preferably polyacrylonitrile of molecular weight $\overline{M}_n$ of 5,000-100,000, of thickness of 10-80 microns, and of molecular weight cut off of 25,000-100,000 and (iii) as a non-porous separating layer at least one layer of polyimine of molecular weight $\overline{M}_n$ of 40,000-100,000 which has been chemically cross-linked with an aliphatic polyhalide.

The composite membranes of this invention may be utilized in various configurations. It is, for example, possible to utilize the composite in a plate-and-frame configuration in which separating layers may be mounted on the porous support layer with the carrier layer.

It is possible to utilize a spiral wound module which includes a non-porous separating layer membrane mounted on a porous support layer and a carrier layer, the assembly being typically folded and bonded or sealed along all the edges but an open edge - to form a bag-like unit which preferably has the separating layer on the outside. A cloth spacer, serving as the permeate or discharge channel is placed within the bag-like unit. The discharge channel projects from the open end of the unit.

There is then placed on one face of the bag-like unit, adjacent to the separating layer, and coterminous therewith, a feed channel sheet - typically formed of a plastic net.

The so-formed assembly is wrapped around a preferably cylindrical conduit which bears a plurality of perforations in the wall - preferably in a linear array which is as long as the width of the bag-like unit. The projecting portion of the discharge channel of the bag-like unit is placed over the performations of the conduit; and the bag-like unit is wrapped around the conduit to form a spiral wound configuration.

It will be apparent that, although only one feed channel is present, the single feed channel in the wound assembly will be adjacent to two faces of the membrane layer. The spiral wound configuration may be formed by wrapping the assembly around the conduit a plurality of times to form a readily handleable unit. The unit is fitted within a shell(in manner comparable to a shell-and-tube heat exchanger) provided with an inlet at one end and an outlet at the other. A baffle-like seal between the inner surface of the shell and the outer surface of the spiral-wound input prevents fluid from bypassing the operative membrane system and insures that fluid enters the system principally at one end. The permeate passes from the feed channel, into contact with the separating layer and thence therethrough, into the permeate channel and thence therealong to and through the perforations in the conduit through which it is withdrawn as net permeate.

In use of the spiral wound membrane, charge liquid is permitted to pass through the plastic net which serves as a feed channel and thence into contact with the non-porous separating membranes. The liquid which does not pass through the membranes is withdrawn as retentate. The liquid or vapor which permeates the membrane passes into the volume occupied by the permeate spacer and through this permeate channel to the perforations in the cylindrical conduit through which it is withdrawn from the system. In this embodiment, it will be apparent that the system may not include a carrier layer.

In another embodiment, it is possible to utilize the system of this invention as a tubular or hollow fibre. In this embodiment, the porous support layer of e.g. polyacrylonitrile may be extruded as a fine tube with a wall thickness of typically 0.001-0.1mm. The extruded tubes are passed through a bath of polyimine polymer in e.g. butanol which is cross-linked and cured in situ. A bundle of these tubes is secured (with an epoxy adhesive) at each end in a header; and the fibres are cut so that they are flush with the ends of the header. This tube bundle is mounted within a shell in a typical shell-and-tube assembly.

In operation, the charge liquid is admitted to the tube side and passes through the inside of the tubes and exits as retentate. During passage through the tubes, permeate passes through the non-porous separating layer and permeate is collected in the shell side.

In this embodiment, it will be apparent that the system may not normally include a carrier layer. In still another embodiment, the porous support layer may be omitted; and the separating layer is extruded and thereafter cross-linked and cured in situ prior to mounting in the headers.

## PERVAPORATION

It is a feature of the non-porous separating layer that it is found to be particularly effective when used in a pervaporation process. In pervaporation, a charge liquid containing a more permeable and a less permeable component is maintained in contact with a non-porous separating layer; and a pressure drop is maintained across that layer. The charge liquid dissolves into the membrane and diffuses therethrough. The permeate which passes through the membrane and exits as a vapor may be recovered by condensing at low temperature or alternatively may be swept away by use of a moving stream of gas. Preferably, the permeate side of the membrane is maintained at a low pressure, typically (5 mm. Hg) 0.65 kPa.

It is a feature of the process of this invention that the novel membrane may be particularly useful in pervaporation processes for dewatering aqueous mixtures of organic oxygenates. It may be possible to utilize the process of this invention to remove water from immiscible mixtures therewith as in the case of ethyl acetate (solubility in water at 15°C of 8.5 parts per 100 parts of water). It will be apparent to those skilled in the art that it may be desirable to separate large quantities of water from partially miscible systems as by decantation prior to utilizing the process of the invention to remove the last traces of water.

The advantages of the instant invention are more apparent when the charge liquid is a single phase homogeneous aqueous solution as is the case for example with isopropanol. The system may also find use in the case of slightly soluble liquids wherein two phases are present (i) water-oxygenate first phase and, as a second phase (ii) either water or oxygenate. Clearly those charge liquids which contain only a small portion of an immiscible second liquid phase may benefit most from the process of this invention. It is also a feature of this invention that it may be particularly useful to separate azeotropes such as isopropanol-water.

The charge organic oxygenates which may be treated by the process of this invention may typically include alcohols, weak acids, ethers, esters, ketones, aldehydes It will be apparent to those skilled in the art that the charge organic oxygenates used should be inert with respect to the separating membrane. Clearly a system wherein the membrane is attacked by the components of the charge liquid will not yield significant separation for any reasonable period of time. Best results may be achieved when treating alcohols (such as isopropanol) or ketones (such as methyl ethyl ketone). Results achieved with acids are generally less satisfactory.

Illustrative alcohols may include ethanol, propanol, i-propanol, n-butanol, i-butanol, t-butanol, amyl, alcohols, hexyl alcohols.

Illustrative ketones may include acetone, methyl ethyl ketone, acetophenone.

Illustrative weak acids may include hexanoic acid, octanoic etc. (When acids are present, preferably the pH of the charge liquid should be above about 4). Typical acids which may be treated by the process of this invention include those having a $pKa \geq ca$ 4.8.

Illustrative esters may include ethyl acetate, methyl acetate, butyl acetate, methyl benzoate, ethylene glycol mono acetate, propylene glycol monostearate.

Illustrative ethers may include tetrahydroforan, diethyl ether, diisopropyl ether.

Illustrative aldehydes may include formaldehyde, acetaldehyde, propionaldehyde.

It is believed that the advantages of this invention are most apparent where the organic oxygenate is a liquid which is infinitely miscible with water - typified by isopropyl alcohol or methyl ethyl ketone.

A typical charge may be an aqueous solution containing 70%-95%, say 85w% isopropanol.

In practice of the pervaporation process of this invention, the charge aqueous organic oxygenate solution typically at 40°C-120°C, say 80°C may be passed into contact with the non-porous separating layer of the membrane of this invention. A pressure drop of about one atmosphere is commonly maintained across the membrane. Typically, the feed or charge side of the membrane is at about atmospheric pressure and the permeate or discharge side of the membrane is at a pressure of (1-50 preferably 2-20, say 10 mm. Hg) 0.13-6.5, preferably 0.26-2.6, say 1.3 kPa.

The permeate which passes through the membrane includes water and a small proportion of the organic oxygenate from the charge liquid. Typically, the permeate contains 80-99.5, say 98w% water. Permeate is recovered in vapor phase.

Performance is judged by the ability of a membrane system to give a permeate containing decreased content of organic oxygenate (from a charge containing a higher content of organic oxygenate and water) with a good flux (kilograms-/meter$^2$-/hour (kg/m$^2$.h)) at a predetermined feed temperature and with a vacuum on the permeate side and a condenser. Compositions falling outside the scope of this invention may be characterized by unsatisfactory separation or unsatisfactory productivity (flux) or both.

Pervaporation may typically be carried out at a flux of about 0.66-7, say 3 kilograms per square meter per hour (kg/m$^2$.h). Typically, the units may show good separation (measured in terms of w% organic oxygenate in the permeate during pervaporation of an aqueous solution of organic oxygenate through a polyethyleneimine separating layer.

It will be noted that as the concentration of oxygenate in the charge increases, the concentration of oxygenate in the permeate increases and the Flux decreases.

The invention will be further described in the following examples wherein, as elsewhere in this specification, all parts are parts by weight unless otherwise stated. An asterisk indicates a control example.

EXAMPLE 1

The selective separating layer is mounted on the porous support layer of a commercially available (under the trademark DUY-L, from Daicel Corp) composite containing a non-woven polyester backing as carrier layer, bearing as porous support layer, a microporous polyacrylonitrile layer of molecular weight cut-off of 40,000.

The separating layer is formed by applying to the porous support layer, a $7.6 \times 10^{-5}$ m thick casting of a 30w% solution in n-butanol of polyethyleneimine, $\overline{M}_n$ of about 60,000, obtained by drying a 30w% aqueous solution of polyethylene imine (available under the trademark Corcat P-600 form Virginia Chemical Co.). The dry material is dissolved in n-butanol. To this solution is added 1,4-dibromobutane (DBB) cross-linking agent. Mole ratio of cross-linking agent to polymer repeat units is 1.67. The coated support is placed in an oven at 125°C for 7 minutes to dry and cure the film.

The membrane made by this method is evaluated in a pervaporation cell to which charge is admitted at 70°C. Permeate pressure is 0.26 kPa (2 mm Hg) at liquid nitrogen temperature.

In this preferred embodiment, the charge solution is an 85w% aqueous solution of isopropanol. The permeate condenser contains an aqueous solution containing only 0.34w% isopropanol (IPA). The Separation Factor (Sep) is 1661 and the Flux is 3.0 kilograms per square meter per hour (kg/m².h) This Flux is more than three times that attained when using a control wherein the separation membrane is a polyvinyl alcohol.

Examples II-VIII

In this series of Examples, the general procedure of Example I is followed, but various conditions were changed including cross-linking agent, mole ratio (R) of cross-linking agent to polyethylene imine, and curing conditions.

In each Example, the polyethylene imine solution is prepared by drying the Corcat™ P-600 aqueous solution on a glass plate at 50°C and dissolving the dried polymer in n-butanol to form a 30w% solution. The casted layer in each case is $7.6 \times 10^{-5}$ m thick.

The mole ratio (R) of cross-linking agent to polyethylene imine is 1.67 in Examples II, III, and VI-VIII. In Example IV, it is 0.83; and in Example V, it is 0.43

TABLE

| Examples | R | Curing Time (min) | Perm %IPA | Flux (kg/m².h) |
|----------|------|-------------------|-----------|----------------|
| II | 1.67 | 7 | 0.34 | 3.0 |
| III | 1.67 | 6 | 0.34 | 2.6 |
| IV | 0.83 | 6 | 0.91 | 1.4 |
| V | 0.43 | 6 | 1.53 | 1.5 |
| VI | 1.67 | 6 | 1.33 | 1.4 |
| VII | 1.67 | 4 | 7.51 | 0.6 |
| VIII | 1.67 | 5 | 1.10 | 1.7 |

From the above Table, the following conclusions may be drawn:

(i) It is possible to obtain good Separation and Flux over the entire range of test conditions;

(ii) Best Separation (0.34w% IPA in the permeate) occurs in Example II when using a film which has been cast and cured at 125°C for 7 minutes from a charge containing a mole ratio of 1,4-dibromobutane to polyethylene imine of 1.67.

(iii) Highest Flux (3.0) is attained under the same conditions.

EXAMPLES IX-XXIII

In this series of Examples, the procedure of Example I is followed except:

(i) the 30% aqueous Corcat™ P-600 solution of polyethylene imine is dried by adding n-butanol and distilling off the water/n-butanol (B) azeotrope. Various amounts of n-hexanol (H) are then added to form solutions of concentrations in w% indicated - the remainder being water;

(ii) the mole ratio (R) of 1,4-dibromobutane to polyethylene imine is as shown;

(iii) the curing temperature is 100°C in Examples XXII-XXIII;

(iv) the time of curing is 6 minutes in Examples IX-XIX, 5 minutes in Example XX, 10 minutes in Example XXII, and 20 minutes in Example XXIII;

(v) the casting thickness is 2.54 x $10^{-5}$ m in Examples XII-XXIII.

TABLE

| Example | Solvent | | R | Selectivity % IPA | Flux kg/m$^2$.h |
|---|---|---|---|---|---|
| | B | H | | | |
| IX | 47 | 33 | 0.67 | 1.60 | 3.2 |
| X | 47 | 33 | 0.33 | 0.33 | 1.5 |
| XI | 47 | 33 | 0.33 | 2.98 | 2.0 |
| XII | 35 | 20 | 0.5 | 4.67 | 2.6 |
| XIII | 35 | | 1.25 | 5.83 | 2.3 |
| XIV | 35 | | 0.67 | 1.32 | 2.9 |
| XV | 35 | | 0.33 | 0.90 | 2.8 |
| XVI | 35 | | 0.17 | 2.48 | 2.9 |
| XVII* | 35 | | no DBB | 2.21 | 2.7 |
| XVIII | 35 | | 0.33 | 3.08 | 3.3 |
| XIX | 35 | | 0.33 | 2.00 | 3.4 |
| XX | 35 | | 3.0 | 2.16 | 3.4 |
| XXI | 33 | | 0.33 | 1.83 | 2.5 |
| XXII | 28 | | 0.33 | 7.50 | 4.2 |
| XXIII | 28 | | 0.33 | 8.94 | 4.1 |

From the above Table, it is apparent that good results are attained across the spectrum of variables.

It will be apparent that although the thermally cross-linked membrane (Example XVII) yielded good Selectivity and Flux, it is possible, by use of chemically cross-linked membranes to attain better Selectivity (Example XIII) or better Flux (Example IX) or both (Example XVI or XVIII). Example XVII is a comparative example and does not belong to the present invention.

EXAMPLES XXIV-XXVII

In this series of Examples, the procedure of Example I is followed except:

(i) the cross-linking agent is 1,4-dibromo-2,3-dihydroxybutane;

(ii) isopropyl alcohol (IPA) is added to the 30w% aqueous solution of polyethylene imine (Corcat™ P-600) in w% indicated;

(iii) the mole ratio (R) of cross-linking agent to polyethylene imine is as shown;

(iv) curing time is 5 minutes and casting film is 2.54 x $10^{-5}$ m thick in Example XXIV, and 10 minutes in Examples XXV-XXVII with a casting 5.08 x $10^{-5}$ m.

TABLE

| Example | Solvent | | R | Selectivty % IPA | Flux kg/m$^2$.h |
|---|---|---|---|---|---|
| | IPA | W | | | |
| XXIV | 30 | 61 | 0.23 | 5.86 | 0.4 |
| XXV | 50 | 35 | 0.48 | 0.56 | 1.5 |
| XXVI | 50 | 35 | 0.23 | 1.67 | 2.3 |
| XXVII | 50 | 35 | 0.48 | 1.25 | 2.0 |

### EXAMPLES XXVIII-XXI

In this series of Examples, the procedure of Example I is followed except:

(i) Isopropyl alcohol (IPA) is added to the 30w% aqueous solution of polyethylene imine, Corcat™ P-600, in weight % indicated;

(ii) the mole ratio of 1,4-dibromobutane to polyethylene imine is as shown;

(iii) the cure time is 10 minutes;

(iv) the casting film is 5.08 x 10$^{-5}$ m thick;

(v) the feed is 4.4w% water, 34.6w% MEK, and 61.0w% Toluene for Examples XXVIII and XXIX and 3.7w% water, 60.6w% MEK, and 35.7w% Toluene for Examples XXX and XXXI.

| Example | Solvent | | R | Selectivity % Water | Flux kg/m$^2$.h |
|---|---|---|---|---|---|
| | IPA | W | | | |
| XXVIII | 50 | 35 | 0.23 | 98.9 | 1.8 |
| XXIX | 50 | 35 | 0.48 | 98.8 | 1.4 |
| XXX | 50 | 35 | 0.23 | 97.7 | 2.7 |
| XXXI | 50 | 35 | 0.48 | 95.5 | 1.7 |

### EXAMPLES XXXII-XXXV

In this series of Examples, the procedure of Example I is followed except:

(i) Isopropyl alcohol (IPA) is added to the 30w% aqueous solution of polyethylene imine, Corcat™ P-600, in weight % indicated;

(ii) the cure time is 10 minutes;

(iii) the casting film is 5.08 x 10$^{-5}$ m thick;

(iv) the cross-linking agent is 1,4-dibromo-2, 3-dihydroxybutane and the mole ratio (R) to polyethylene imine is as indicated;

(v) the feed is 4.4w% water, 34.6w% MEK, and 61.0w% Toluene for Examples XXXII and XXXII and 3.7w% water, 60.6w% MEK, 35.7w% Toluene for Examples XXXIV and XXXV.

| Example | Solvent | | R | Selectivity % Water | Flux kg/m$^2$.h |
|---|---|---|---|---|---|
| | IPA | W | | | |
| XXXII | 50 | 35 | 0.23 | 99.5 | 1.5 |
| XXXIII | 50 | 35 | 0.48 | 94.2 | 1.3 |
| XXXIV | 50 | 35 | 0.23 | 98.1 | 2.3 |
| XXXV | 50 | 35 | 0.48 | 96.3 | 1.5 |

Results comparable to Example I may be attained if the charge liquid is:

TABLE

| Example | Charge Liquid |
|---------|---------------|
| XXXVI | 75 W% isopropyl alcohol<br>25 W% water |
| XXXVII | 90 W% n-hexanoic acid<br>10 W% water |
| XXXIII | 95 W% methyl acetate<br>5 W% water |
| XXXIVX | 85 W% tetrahydrofuran<br>15 W% water |

Results comparable to Example I may be attained if the cross-linking agent is:

## TABLE

| Example | Cross-linking Agent |
|---------|---------------------|
| XL | 1,5-dibromo-n-pentane |
| XLI | 1,6-dibromo-hexane |
| XLII | 1,4-dichloro-n-butane |

## Claims

1. A method of concentrating an aqueous charge mixture of an organic oxygenate which comprises passing said aqueous charge into contact with the high pressure side of a non-porous membrane layer of a polyimine polymer which has firstly been cross-linked with an aliphatic polyhalide and which has secondly been thermally crosslinked maintaining a pressure drop across said layer whereby at least a portion of said water and a lesser portion of organic oxygenate from said aqueous charge pass by pervaporation through said separating layer as a lean mixture containing more water and less organic oxygenate than are present in said aqueous charge, leaving a rich liquid containing less water and more organic oxygenate than are present in said aqueous charge.

2. A method as claimed in Claim 1 wherein said polyimine is of formula

   $H_2NR''[N-R'']_n - NH_2$

   wherein $R''$ is an alkylene, aralkylene, cycloalkylene, arylene, or alkarylene hydrocarbon group and $\underline{n}$ is the number of recurring groups in the polymer chain.

3. A method as claimed in Claim 2 wherein $R''$ is ethylene - $CH_2CH_2$ -.

4. A method as claimed in any one of Claims 1 to 3 wherein said aliphatic polyhalide contains 2-12 carbon atoms.

5. A method as claimed in any one of Claims 1 to 4 wherein said aliphatic polyhalide is a dihalide.

6. A method as claimed in any one of Claims 1 to 5 wherein said organic oxygenate is an alcohol.

7. A non-porous membrane comprising a separating layer of a polyimine polymer which has firstly been chemically cross-linked with an aliphatic polyhalide and which has secondly been thermally crosslinked

8. A non-porous membrane as claimed in Claim 7 wherein said polyimine is of formula

$H_2NR''[N-R'']_n - NH_2$

wherein R'' is an alkylene, aralkylene, cycloalkylene, arylene, or alkarylene hydrocarbon group and $\underline{n}$ is the number of recurring groups in the polymer chain.

9. A non-porous membrane as claimed in Claim 8 wherein said R'' is ethylene $-CH_2CH_2-$.

10. A non-porous membrane as claimed in Claim 7 comprising a separating layer of polyethylene imine, of molecular weight $\overline{M}_n$ of 40,000-100,000, which has been cross-linked with a 1,4-dibromobutane, and thereafter thermally crosslinked

11. The use of a membrane as claimed in any one of Claims 7 to 10 in separating water from an aqueous charge mixture of organic oxygenate by pervaporation

12. A method of preparing a non-porous membrane separating layer of a polyimine polymer of molecular weight $\overline{M}_n$ of 40,000-100,000 which comprises casting a solution of said polyimine polymer to which has been added an aliphatic polyhalide crosslinking agent, followed by thermally crosslinking the casted membrane layer, thereby forming a cured chemically cross-linked membrane layer.

**Patentansprüche**

1. Ein Verfahren zum Aufkonzentrieren einer wäßrigen Chargenmischung einer organischen Sauerstoffverbindung, welches umfaßt, daß besagte wäßrige Charge in Kontakt mit der Hochdruckseite einer nicht-porösen Membranschicht aus einem Polyiminpolymer, das erstens mit einem aliphatischen Polyhalogenid quervernetzt worden ist und das zweitens thermisch quervernetzt worden ist, geleitet und ein Druckabfall über besagte Schicht gehalten wird, wodurch wenigstens ein Teil von besagtem Wasser und ein geringerer Teil von organischer Sauerstoffverbindung aus besagter wäßrigen Charge durch Pervaporation durch besagte Trennschicht als eine magere Mischung hindurchgehen, die mehr Wasser und weniger organische Sauerstoffverbindung enthält, als in besagter wäßrigen Charge vorhanden sind, wobei eine reiche Flüssigkeit zurückbleibt, die weniger Wasser und mehr organische Sauerstoffverbindung enthält, als in besagter wäßrigen Charge vorhanden sind.

2. Ein Verfahren nach Anspruch 1, wobei besagtes Polyimin die Formel besitzt

$H_2NR''[N-R'']_n - NH_2$

worin R'' eine Alkylen-, Aralkylen-, Cycloalkylen-, Arylen- oder Alkarylenkohlenwasserstoffgruppe ist und $\underline{n}$ die Anzahl von sich wiederholenden Gruppen in der Polymerkette ist.

3. Ein Verfahren nach Anspruch 2, wobei R'' Ethylen - $CH_2CH_2$ -ist.

4. Ein Verfahren nach einem der Ansprüche 1 bis 3, wobei besagtes aliphatische Polyhalogenid 2-12 Kohlenstoffatome enthält.

5. Ein Verfahren nach einem der Ansprüche 1 bis 4, wobei besagtes aliphatische Polyhalogenid ein Dihalogenid ist.

6. Ein Verfahren nach einem der Ansprüche 1 bis 5, wobei besagte organische Sauerstoffverbindung ein Alkohol ist.

7. Eine nicht-poröse Membran, die eine Trennschicht aus einem Polyiminpolymer umfaßt, das erstens chemisch mit einem aliphatischen Polyhalogenid quervernetzt worden ist und das zweitens thermisch quervernetzt worden ist.

**8.** Eine nicht-poröse Membran nach Anspruch 7, wobei besagtes Polyimin die Formel besitzt

$$H_2NR''[N-R'']_n - NH_2$$

worin R'' eine Alkylen-, Aralkylen-, Cycloalkylen-, Arylen- oder Alkarylenkohlenwasserstoffgruppe ist und $\underline{n}$ die Anzahl von sich wiederholenden Gruppen in der Polymerkette ist.

**9.** Eine nicht-poröse Membran nach Anspruch 8, wobei R'' Ethylen - $CH_2CH_2$ - ist.

**10.** Eine nicht-poröse Membran nach Anspruch 7, die eine Trennschicht aus Polyethylenimin, mit einem Molekulargewicht $\overline{M}_n$ von 40.000-100.000, umfaßt, die mit einem 1,4-Dibrombutan quervernetzt und danach thermisch quervernetzt worden ist.

**11.** Die Verwendung einer Membran nach einem der Ansprüche 7 bis 10 zur Abtrennung von Wasser aus einer wäßrigen Chargenmischung von organischer Sauerstoffverbindung durch Pervaporation.

**12.** Ein Verfahren zur Herstellung einer nicht-porösen Membran-Trennschicht aus ein Polyiminpolymer mit einem Molekulargewicht $\overline{M}_n$ von 40.000-100.000, welches umfaßt, daß eine Lösung von besagtem Polyiminpolymer, zu dem ein Vernetzungsmittel auf der Basis von aliphatischem Polyhalogenid zugesetzt worden ist, gegossen wird, gefolgt von thermischer Quervernetzung der gegossenen Membranschicht, wodurch eine ausgehärtete, chemisch quervernetzte Membranschicht gebildet wird.

**Revendications**

**1.** Procédé de concentration d'un mélange de charge aqueuse d'un oxygénat organique, qui comprend le fait d'amener cette charge aqueuse au contact du côté à haute pression d'une couche de membrane non poreuse d'une polyimine polymère qui a en premier lieu été réticulée avec un polyhalogénure aliphatique et qui a en second lieu été thermiquement réticulée, et de maintenir une chute de pression à travers cette couche de telle sorte qu'au moins une partie de cette eau et une partie moindre d'oxygénat organique provenant de cette charge aqueuse traversent par pervaporation cette couche séparatrice sous la forme d'un mélange pauvre contenant davantage d'eau et moins d'oxygénat organique qu'il n'y en a dans cette charge aqueuse, laissant un liquide riche contenant moins d'eau et davantage d'oxygénat organique qu'il n'y en a dans cette charge aqueuse.

**2.** Procédé selon la revendication 1, dans lequel cette polyimine répond à la formule :

$$H_2NR''[N-R'']_n - NH_2$$

dans laquelle R'' est un groupe hydrocarboné alkylène, aralkylène, cycloalkylène, arylène ou alcarylène et n est le nombre de motifs récurrents dans la chaîne du polymère.

**3.** Procédé selon la revendication 2, dans lequel R'' est l'éthylène -$CH_2CH_2$-.

**4.** Procédé selon l'une quelconque des revendications 1 à 3, dans lequel ce polyhalogénure aliphatique contient 2 à 12 atomes de carbone.

**5.** Procédé selon l'une quelconque des revendications 1 à 4, dans lequel ce polyhalogénure aliphatique est un dihalogénure.

**6.** Procédé selon l'une quelconque des revendications 1 à 5, dans lequel cet oxygénat organique est un alcool.

**7.** Membrane non poreuse comprenant une couche de séparation d'une polyimine polymère qui a en premier lieu été réticulée chimiquement avec un polyhalogénure aliphatique et qui a en second lieu été réticulée thermiquement.

**8.** Membrane non poreuse selon la revendication 7, dans laquelle cette polyimine répond à la formule

EP 0 496 496 B1

H$_2$NR''[N-R'']$_n$ - NH$_2$

dans laquelle R'' est un groupe hydrocarboné alkylène, aralkylène, cycloalkylène, arylène ou alcarylène et n est le nombre de motifs dans la chaîne du polymère.

9. Membrane non poreuse selon la revendication 8, dans laquelle ce R'' est l'éthylène -CH$_2$CH$_2$-.

10. Membrane non poreuse selon la revendication 7, comprenant une couche séparatrice de polyéthylèneimine, d'une masse moléculaire $\overline{M}_n$ de 40 000 à 100 000, qui a été réticulée avec un 1,4-dibromobutane, puis réticulée thermiquement.

11. Utilisation d'une membrane selon l'une quelconque des revendications 7 à 10 pour séparer l'eau d'un mélange de charge aqueuse d'oxygénat organique par pervaporation.

12. Procédé de préparation d'une couche séparatrice de membrane non poreuse d'une polyimine polymère d'une masse moléculaire $\overline{M}_n$ de 40 000 à 100 000, qui comprend (1) le fait de couler une solution de cette polyimine polymère, (2) à laquelle a été ajouté un polyhalogénure aliphatique comme agent de réticulation, après quoi on réticule thermiquement la couche de membrane coulée, en formant une couche de membrane durcie réticulée chimiquement durcie.

14